(19) Europäisches Patentamt

European Patent Office

Office européen des brevets

(11) **EP 0 721 486 B1**

(12) **FASCICULE DE BREVET EUROPEEN**

(45) Date de publication et mention
de la délivrance du brevet:
**20.02.2002  Bulletin 2002/08**

(51) Int Cl.[7]: **C09D 17/00**, B01F 17/00

(21) Numéro de dépôt: **94928923.5**

(22) Date de dépôt: **28.09.1994**

(86) Numéro de dépôt international:
**PCT/FR94/01132**

(87) Numéro de publication internationale:
**WO 95/09207 (06.04.1995 Gazette 1995/15)**

(54) **UTILISATION DE COPOLYMERES-BLOCS ACRYLIQUES COMME AGENTS MOUILLANTS ET/OU DISPERSANTS DES PARTICULES SOLIDES ET DISPERSIONS EN RESULTANT**

VERWENDUNG VON ACRYLBLOCKCOPOLYMERE ALS NETZMITTEL UND/ODER DISPERGIERMITTEL FÜR FESTSTOFFTEILCHEN UND DIE DARAUS HERGESTELLTEN DISPERSIONEN

USE OF ACRYLIC BLOCK COPOLYMERS AS WETTING AND/OR DISPERSING AGENTS FOR SOLID PARTICLES AND RESULTING DISPERSIONS

(84) Etats contractants désignés:
**BE CH DE ES FR GB IT LI NL**

(30) Priorité: **30.09.1993  FR 9311691**

(43) Date de publication de la demande:
**17.07.1996  Bulletin 1996/29**

(73) Titulaire: **LVMH RECHERCHE
92752 Nanterre (FR)**

(72) Inventeurs:
• **HOSOTTE-FILBERT, Claude
F-90000 Belfort (FR)**
• **TONDEUR, Carole
F-68100 Mulhouse (FR)**
• **RIESS, Henri-Gérard
F-68200 Mulhouse (FR)**

• **MEYBECK, Alain
F-92400 Courbevoie (FR)**
• **TRANCHANT, Jean-François
F-45760 Boigny-sur-Bionne (FR)**

(74) Mandataire: **Giraud, Françoise et al
Cabinet Beau de Loménie 158, rue de l'Université
75340 Paris Cedex 07 (FR)**

(56) Documents cités:
DE-A- 4 218 734          FR-A- 2 185 656
GB-A- 2 005 697

• MACROMOLECULES, vol.24, 1991, US pages 4997 - 5000 VARSHNEY ET AL 'ANIONIC POLYMERIZATION OF ACRYLIC MONOMERS' cité dans la demande

**Description**

La présente invention concerne une nouvelle utilisation de copolymères-blocs acryliques comme agents mouillants et/ou dispersants des particules solides ainsi que des dispersions de particules solides contenant ces copolymères-blocs.

L'utilisation de tensioactifs macromoléculaires pour le traitement des surfaces en milieux non aqueux a fait l'objet de nombreuses études, en particulier d'études de laboratoire visant à préciser le mécanisme et la cinétique de la fixation des polymères à la surface de poudres particulières.

A titre d'exemples de telles études, on citera :

- des études utilisant différents copolymères-blocs désignés symboliquement par A-b-B, A et B représentant les deux blocs et b indiquant qu'il s'agit de copolymères-blocs ;
- les travaux de Du Pont de Nemours sur une silice dispersée dans l'isopropanol à l'aide de copolymères-blocs à base de méthacrylate de butyle et de méthacrylate de diméthylaminoéthyle. (Wu, Yokoyama, Setterquist, Polym. J. (91) Vol. 23 page 409) ;
- les travaux de Molau et Richardson sur l'oxyde de titane dispersé par des copolymères polystyrène-b-polybutadiène (Adv. Chem. Ser. (71), vol. 99, page 379) ;
- diverses études sur des silices en présence de copolymères polystyrène-b-polyvinylpyridine dans le toluène par exemple : J.F. Tassin, R.L. Siemens, Wing T. Tang, G. Hadziioannou, J.D. Swales, B. A. Smith, J. Phys. Chem. 1989, 93, 2106-2111 ;
- les travaux de Leemans, Fayt, Teyssié, Uijtterhoeven, Polymer (90), Vol. 31, page 108 sur la dispersion du noir de carbone par des copolymères polystyrène-b-polyméthacrylate de stéaryle ;
- ainsi que des études utilisant des copolymères de type statistique : on citera par exemple l'étude de l'adsorption sur $TiO_2$ de copolymères amphiphiles statistiques présentant un squelette constitué de méthacrylate de méthyle et d'acrylonitrile et portant des greffons heptamères d'acide hydroxystéarique publiée dans J. of Colloid and Interface Sci., 26, 214-221 (1968).

On connaît des copolymères-blocs entre l'acide acrylique ou méthacrylique (symbolisé par le bloc A) et l'acrylate ou le méthacrylate d'alkyle (symbolisé par le bloc B), la chaîne alkyle étant comprise entre C1 et C10. Ces copolymères peuvent être du type dibloc (A-b-B) ou tribloc (A-b-B-b-A ou B-b-A-b-B). Dans la suite, ils sont symbolisés par AB qu'ils soient di- ou triblocs. Leur synthèse est en particulier décrite par :

- SK Varshney, C. Jacobs, J.-P. Hautekcer, P. Bayard, R. Jérôme, R. Fayt, P. Teyssié, Macromolecules, vol. 24, p. 4997-5000 (1991) pour des copolymères di- et triblocs entre l'acide acrylique et le méthacrylate de méthyle ;
- T.E. Long, C.D. DePorter, N. Patel, D.W. Dwight, G.L. Wilkes, J.E. McGrath, Polym. Prep., vol. 28(2), p. 214-216 (1987) pour des copolymères diblocs entre l'acide méthacrylique et le méthacrylate de 2-éthyl hexyle ;
- C.D. DePorter, T.E. Long, L.N. Venkateshwaran, G.L. Wilkes, J.E. McGrath, Polym. Prep., vol. 29(1), p. 343-345 (1988) pour des copolymères triblocs entre l'acide méthacrylique et le méthacrylate de 2-éthyl hexyle ;
- C.D. DePorter, L.N. Venkateshwaran, G.A. York, G.L. Wilkes, J.E. McGrath, Polym. Prep., vol. 30(1), p. 201-203 (1989) pour des copolymères di- et triblocs entre l'acide méthacrylique et le méthacrylate de n-hexyle.

Le procédé décrit permet de faire varier la nature et la longueur de chacun des blocs polymères en ajustant la concentration en amorceur et en monomères.

La demanderesse a maintenant découvert que cette famille de copolymères acryliques, symbolisés dans la suite par AB, et en particulier PAA-b-PMMA, pouvait être avantageusement utilisée pour modifier l'énergie de surface de particules solides vis-à-vis de liquides organiques, ce qui conduit à leur utilisation comme agents mouillants et/ou dispersants de ces particules dans des milieux organiques pour l'obtention de dispersions stables. Ainsi, l'utilisation de ces copolymères-blocs permet de résoudre avantageusement le problème de la formation d'agrégats de particules lorsque l'on cherche à former des dispersions stables de ces particules dans un solvant.

Ainsi, selon une de ses caractéristiques essentielles, l'invention concerne l'utilisation des copolymères-blocs symbolisés par AB de l'acide acrylique ou méthacrylique, le(s) bloc(s) correspondant(s) étant symbolisé(s) par A, et de l'acrylate ou du méthacrylate d'alkyle avec la chaîne alkyle comprise entre $C_1$ et $C_{10}$, le(s) bloc(s) correspondant (s) étant symbolisé(s) par B, comme agents mouillants et/ou dispersants des particules solides dans un milieu essentiellement organique constitué d'un solvant ou mélange de solvants solubilisant le groupe B.

Par milieu essentiellement organique, on entend, dans la présente description, un milieu contenant moins de 5 % d'eau et de préférence moins de 1 % d'eau.

Les études de la demanderesse l'ont conduite pour les différents copolymères de la famille, en fonction de la nature du milieu dans lequel on souhaite disperser la poudre ainsi que de la nature de la poudre :

- d'une part, à déterminer le rendement d'adsorption du copolymère, défini comme la quantité de copolymère "fixé" par rapport à la quantité engagée. Par copolymère "fixé", on entend copolymère adsorbé par des liaisons physiques sur la surface de la particule solide,
- et, d'autre part, à déterminer l'efficacité de l'agent mouillant.

Les études de la demanderesse l'ont amenée, en particulier, à mettre en évidence l'existence d'un palier dans les courbes, dites isothermes d'adsorption, représentant le taux d'adsorption du copolymère en fonction de la concentration initiale du copolymère à une température donnée.

L'efficacité de l'agent mouillant et/ou dispersant sera caractérisée par la quantité minimale de copolymère à engager pour atteindre le palier de l'isotherme d'adsorption et recouvrir toute la surface de la particule.

Les études de la demanderesse l'ont conduite à mettre en évidence la réduction significative de l'énergie de surface des particules solides, de la quantité d'agrégats formés entre les particules et de leur taille en présence de copolymère-bloc AB dans le milieu.

La demanderesse a également mis en évidence que les copolymères acryliques AB constituaient de remarquables agents stabilisants des dispersions de particules solides dans les milieux organiques et permettaient en particulier d'obtenir des dispersions dont le surnageant ne devient pas limpide avant au moins 24 h pour des teneurs en copolymères correspondant au palier de l'isotherme d'adsorption.

Les copolymères-blocs de type AB définis précédemment sont avantageusement des copolymères diblocs symbolisés par A-b-B.

Il peut également s'agir de copolymères triblocs de type A-b-B-b-A ou B-b-A-b-B.

Selon une variante particulièrement avantageuse de l'invention, on choisit des copolymères diblocs de l'acide acrylique (symbolisé par AA) et de méthacrylate de méthyle (symbolisé par MMA) représentés par PAA-b-PMMA.

Les particules solides peuvent être de tout type et ont avantageusement des dimensions comprises entre quelques nanomètres et quelques millimètres, de préférence entre 50 nm et 100 $\mu$m.

Il pourra s'agir en particulier, de particules minérales, en particulier de particules constituées d'oxydes métalliques, par exemple d'oxyde de titane, de zinc, de fer, de zirconium, ou de silice, d'alumine, de carbonate de calcium.

Ces pigments peuvent être des pigments fixés ou non sur un support minéral ou organique.

L'invention est tout particulièrement applicable aux pigments minéraux.

A titre d'exemple de pigments, on citera l'oxyde de titane, l'oxyde de fer, l'oxyde de zinc, les colorants azoïques sous forme de laque de calcium ou de baryum.

Les supports minéraux peuvent être constitués d'alumine, de silice, ou encore de $TiO_2$.

Les copolymères-blocs acryliques AB utilisés selon l'invention comme agents dispersants et/ou mouillants des particules solides en milieu organique, en particulier les copolymères PAA-b-PMMA ont avantageusement une masse molaire moyenne en nombre ($M_n$) comprise entre 2 000 et 500 000, de préférence entre 2 000 et 100 000.

Le bloc A, en particulier PAA, de ces copolymères représente de 5 à 95 % en poids du copolymère pour obtenir un effet mouillant.

Pour obtenir en outre un effet dispersant conduisant à une dispersion stable, le bloc A représente de préférence moins de 50 % en poids du copolymère.

Le solvant organique utilisé pour réaliser le traitement des particules peut être :

- tout solvant des deux séquences A et B,
- tout solvant de la séquence B, non solvant de la séquence A,
- tout mélange de solvants qui solubilise les deux séquences,
- tout mélange de solvants qui solubilise la séquence B, mais ne solubilise pas la séquence A.

A titre d'exemple de solvants utilisables dans le cas des copolymères PAA-b-PMMA :

- le dioxane, la diméthylformamide, le diméthylsulfoxyde comme solvant des deux séquences,
- tout solvant de la séquence PMMA, soit par exemple des esters tels que l'acétate de méthyle, d'éthyle, de propyle, de butyle ou d'amyle, des cétones tels que l'acétone, la méthyl éthyl cétone, la méthyl butyl cétone ou la cyclohexanone, des solvants chlorés tels que le chloroforme ou le dichlorométhane, des aromatiques, par exemple le toluène ou le xylène, l'acide acétique,
- des mélanges des solvants précédents ou mélanges d'un solvant du PMMA avec un non solvant du PMMA dans des proportions gardant cette séquence soluble, comme par exemple un mélange acétate de butyle/éthanol ou isopropanol contenant au moins 50 % d'acétate de butyle.

Selon un autre de ses aspects, l'invention concerne un procédé pour préparer des dispersions stables de particules solides en présence d'un agent dispersant dans un milieu essentiellement organique contenant moins de 5% et de préférence moins de 1% d'eau, caractérisé en ce qu'on utilise à titre d'agent dispersant un copolymère-bloc du type AB tel que défini dans la revendication 1 et à titre de milieu essentiellement organique, un solvant ou un mélange de solvants, solvant dudit bloc B.

Les particules utilisables pour préparer les dispersions selon l'invention sont toutes les particules telles qu'elles ont été précédemment définies, en particulier les particules solides de dimensions comprises entre quelques nanomètres et quelques millimètres, de préférence entre 50 nm et 100 μm.

L'invention s'applique tout particulièrement à la dispersion des particules solides minérales, en particulier des particules d'oxydes métalliques et plus particulièrement des pigments minéraux supportés ou non sur un support organique ou minéral.

Parmi les dispersions de l'invention, on citera tout particulièrement les dispersions de particules d'oxyde de titane.

Les copolymères utilisables pour préparer les dispersions selon l'invention sont tous les copolymères-blocs de type AB définis précédemment en particulier les copolymères PAA-b-PMMA, avantageusement ceux dont la masse molaire moyenne en nombre est comprise entre 2 000 et 500 000, de préférence entre 2 000 et 100 000.

Le bloc A, en particulier PAA, représente de *5* à 95 % en poids du copolymère-bloc, de préférence moins de 50 % en poids dudit copolymère-bloc.

Les dispersions selon l'invention contiennent de 0,1 à 30 % en poids de copolymère-bloc par rapport aux particules solides, de préférence de 0,5 % à 15 % (ceci est fonction de la surface spécifique des particules solides).

Les milieux essentiellement organiques utilisés pour préparer les dispersions selon l'invention peuvent être :

- tout solvant des deux séquences A et B,
- tout solvant de la séquence B, non solvant de la séquence A,
- tout mélange de solvants qui solubilise les deux séquences,
- tout mélange de solvants qui solubilise la séquence B, mais ne solubilise pas la séquence A.

A titre d'exemple de solvants utilisables dans le cas des copolymères PAA-b-PMMA :

- le dioxane, la diméthylformamide, le diméthylsulfoxyde comme solvant des deux séquences,
- tout solvant de la séquence PMMA, soit par exemple des esters tels que l'acétate de méthyle, d'éthyle, de propyle, de butyle ou d'amyle, des cétones tels que l'acétone, la méthyl éthyl cétone, la méthyl butyl cétone ou la cyclohexanone, des solvants chlorés tels que le chloroforme ou le dichlorométhane, des aromatiques, par exemple le toluène ou le xylène, l'acide acétique,
- des mélanges des solvants précédents ou mélanges d'un solvant du PMMA avec un non solvant du PMMA dans des proportions gardant cette séquence soluble, comme par exemple un mélange acétate de butyle/éthanol ou isopropanol contenant au moins 50 % d'acétate de butyle.

On choisit avantageusement, comme solvant ou mélange des solvants, les esters, par exemple l'acétate de butyle, de propyle ou d'éthyle ou les mélanges esters/alcools constitués majoritairement d'esters.

Un solvant particulièrement utile pour préparer les dispersions de l'invention est l'acétate de butyle. On utilise également avantageusement les mélanges acétate de butyle/éthanol contenant au moins 50 % d'acétate de butyle.

L'invention s'applique tout particulièrement à la dispersion des particules d'oxyde de titane, en particulier des particules de dimensions comprises entre 50 nm et 1 μm.

Dans la pratique, on détermine le rendement d'adsorption de la façon suivante : une masse $m_s$ de solution de concentration initiale Ci en copolymère est mise en contact avec une masse $m_c$ de particules de surface spécifique S. Après adsorption, on détermine, après élimination des particules par centrifugation, la nouvelle concentration (Ce) de la solution. Le rendement (rdt) est donné par la formule

$$Rdt = (Ci-Ce)/Ci \times 100 \ (\%)$$

Le taux d'adsorption (Γ) est donné, lui, par :

$$\Gamma = [(Ci-Ce) \times m_s]/m_c \times S$$

Les essais montrent qu'une faible quantité de copolymères suffit pour disperser correctement une charge. A titre d'exemple, dans le cas d'une masse de 1 g de poudre d'oxyde de titane de 10 m$^2$/g et lorsque le solvant est l'acétate de butyle, il faut environ 40 mg de copolymère PAA-b-PMMA de masse molaire moyenne en nombre ($M_n$) de

9 900 contenant 22 % en masse de PAA pour atteindre le palier de l'isotherme d'adsorption à 20°C.

La dispersibilité est évaluée à partir des mesures de tailles des particules, par exemple à l'aide d'un appareil de type Coulter LS130.

Un avantage particulier des copolymères-blocs utilisés comme dispersants selon l'invention est qu'ils conduisent à des dispersions stables.

La stabilisation des dispersions est appréciée soit par mesure de la vitesse de sédimentation des suspensions en fonction du temps, soit par suivi des variations de l'absorbance du surnageant en fonction de la durée d'une centrifugation.

Un autre avantage des dispersions selon l'invention est que, lorsqu'il se produit une décantation au cours du temps, le dépôt formé reste facilement redispersable dans le milieu après agitation, le copolymère recouvrant totalement la surface de la particule, empêchant ainsi son agrégation.

Un autre avantage des dispersions selon l'invention est que, lorsqu'elles sont séchées à température ambiante, la poudre récupérée peut être facilement redispersée par la suite dans le solvant.

Selon une autre de ses caractéristiques essentielles, l'invention concerne également toute composition renfermant les dispersions précédemment décrites. Elle concerne tout particulièrement les compositions contenant des dispersions de pigments destinées au domaine de la peinture ou de la cosmétique.

Un avantage particulier des dispersions selon l'invention dans lesquelles ce sont les séquences B, en particulier les séquences PMMA qui sont stabilisatrices est qu'elles sont compatibles avec la nitrocellulose, ce qui permet leur utilisation en particulier dans la préparation des vernis à ongles.

Les exemples qui suivent sont donnés à titre purement illustratif de l'invention.


Exemples


Exemple 1 : Dispersion d'une poudre d'oxyde de titane

On utilise comme copolymère un copolymère PAA-b-PMMA de $M_n$ = 9 900 contenant 22 % de PAA.

Le solvant est de l'acétate de butyle.

Le copolymère est mis en solution dans l'acétate de butyle. On ajoute ensuite 3 g d'oxyde de titane de surface spécifique égale à 10 m$^2$/g à 10 g de solution et on agite pendant 24 h.

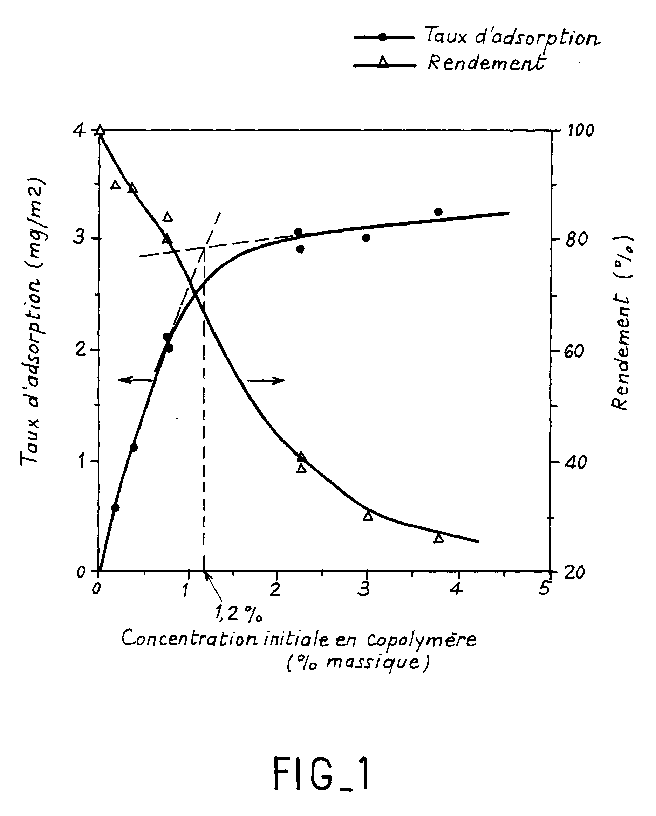

La figure 1 donne le taux d'adsorption et le rendement d'adsorption en fonction de la concentration initiale en copolymère, exprimée en pourcentages massiques. Le rendement, Rdt, est donné par la relation :

$$Rdt = (Ci-Ce)/Ci \times 100 \ (\%)$$

où Ci représente la concentration initiale en copolymère et Ce la concentration de la solution après mise en contact de la solution avec les particules solides et séparation des particules solides par centrifugation.

Le taux d'adsorption est donné par

$$\Gamma = [(Ci-Ce) \ m_s]/m_c S$$

où $m_s$ est la masse de solution et $m_c$ la masse de TiO$_2$ de surface spécifique S.

Dans le cas présent :

$m_s$ = 10 g
S = 10 m$^2$/g
$m_c$ = 3 g.


Le tracé de la courbe de la figure 1 donnant le taux d'adsorption en fonction de la concentration initiale en copolymère à 20°C (isotherme d'adsorption) montre clairement l'existence d'un palier dont on détermine le début par tracé des tangentes comme indiqué sur la figure 1.

Dans le cas particulier de cet exemple, le début du palier lu sur la courbe correspond à une concentration initiale en copolymère de 1,2 % en masse.

Le calcul montre alors qu'il faut dans ce cas 40 mg pour stabiliser et disperser 1 g de TiO$_2$.

Exemple 2 : Mise en évidence de la modification de l'énergie de surface

Pour le TiO$_2$ traité par des copolymères PAA-b-PMMA, suivant le mode opératoire décrit dans l'exemple 1, la modification de l'énergie de surface est estimée par le test de Stevens tel qu'il est décrit par P. Stevens, L. Gypen et R. Jennen-Bartholomeussen dans "Wettability of powders" Farmaceutisch Tijdschrift voor Belgïe, 51e jaargang, nummer 2, maart-april 1974.

Avant traitement, le TiO$_2$ présente une énergie de surface d'environ 70 mJ/m$^2$ donc un caractère hydrophile marqué. Après adsorption du copolymère (Ci=3 %) on observe une forte diminution de l'énergie de surface jusqu'à une valeur d'environ 40 mJ/m$^2$.

Ces observations sont confirmées par chromatographie inverse en phase gazeuse à dilution infinie.

Exemple 3 : Mise en évidence de la dispersion de particules de TiO$_2$

La dispersibilité est évaluée à partir de mesures de tailles de particules à l'aide du Coulter LS130.

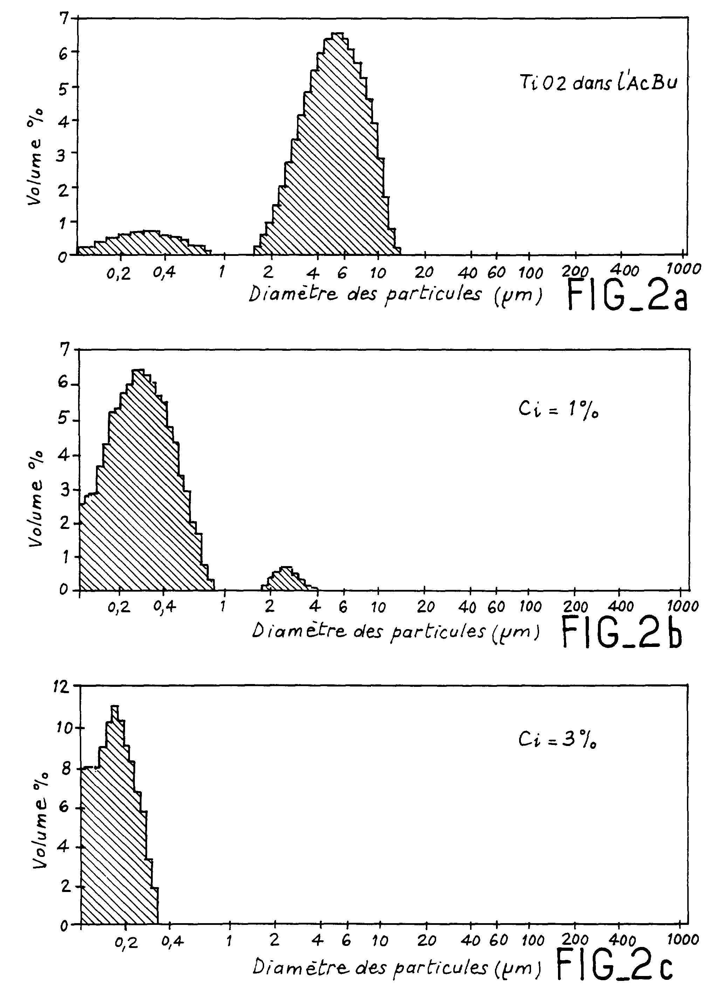

Les figures 2a, 2b et 2c présentent les diagrammes d'analyses granulométriques obtenus à l'aide de l'appareil Coulter LS130 pour du TiO$_2$ à différents stades du traitement pour le même exemple que l'isotherme d'adsorption donnée à l'exemple 1. Le copolymère permet de réduire de façon considérable les agrégats jusqu'à les faire disparaître lorsque le recouvrement du TiO$_2$ est total (c'est-à-dire pour des concentrations initiales en polymère correspondant au palier de l'isotherme).

Pour ce type d'exemple, un très bon dispersant doit réduire la quantité d'agrégats à une valeur inférieure à 1 %.

La figure 2a montre la prédominance des agrégats en l'absence de copolymère dans la solution.

La figure 2b, qui correspond à une concentration initiale Ci en copolymère de 1 % dans l'acétate de butyle, soit 3,33 % par rapport au TiO$_2$, montre que, même avant le palier, il y a déjà une nette régression de la proportion d'agrégats.

La figure 2c qui concerne des conditions correspondant au palier de l'isotherme, en l'occurrence une concentration initiale Ci de 3 %, montre la complète disparition des agrégats.

Exemple 4 : Mise en évidence de la stabilisation d'une dispersion de particules

On étudie la vitesse de sédimentation des trois suspensions étudiées dans l'exemple 3, par mesure de la hauteur de l'interphase en fonction du temps.

La figure 3 donne la hauteur de l'interphase (en cm) des suspensions dans des tubes en fonction du temps pour chacune des suspensions correspondant respectivement à :

- une suspension de TiO$_2$ en l'absence de copolymère-bloc (TiO$_2$ non traité),
- une suspension avant le palier de l'isotherme (Ci = 1 %),
- une suspension sur le palier de l'isotherme (Ci = 3 %).

Exemple 5 : Mise en évidence de la stabilisation

Cet exemple met en évidence, lui aussi, la stabilisation de la même suspension de poudre de TiO$_2$ que dans les exemples précédents par étude de la variation de l'absorbance du surnageant en fonction de la durée d'une centrifugation de 120 rpm (Shimadzu SA - CP3).

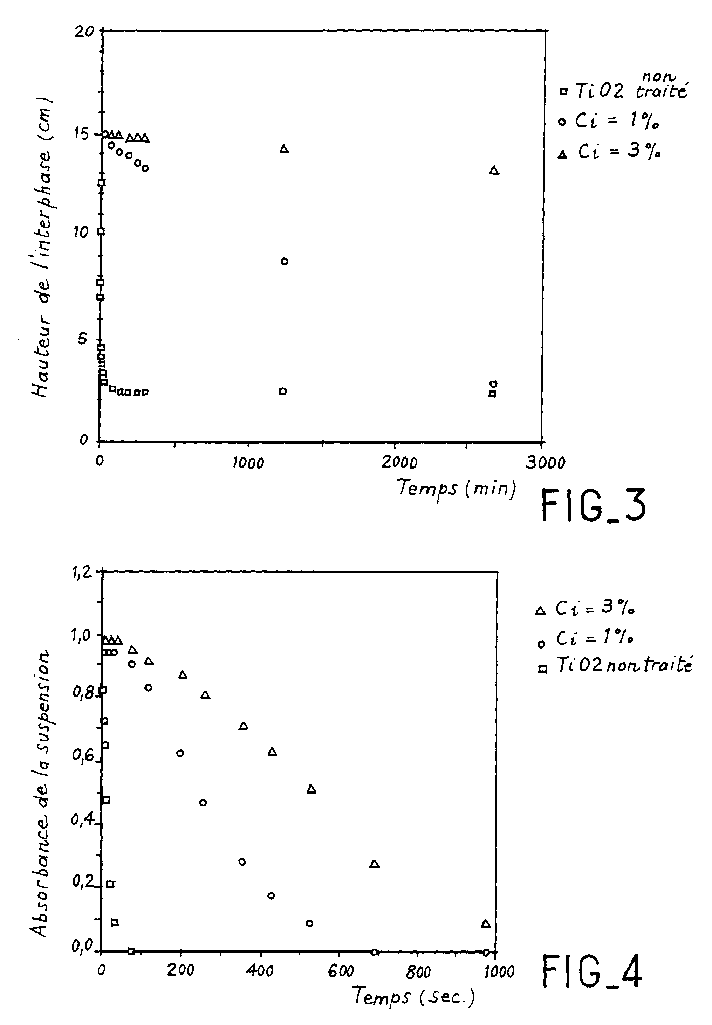

La figure 4 donne les résultats obtenus d'une part avec TiO$_2$ dans l'acétate de butyle en l'absence de copolymère-bloc, d'autre part en présence de PAA-b-PMMA avant et sur le palier de l'isotherme, dans les mêmes conditions que dans les exemples précédents (Ci = 1 % et Ci = 3 %).

Exemple 6: Mise en évidence de la stabilisation en présence de nitrocellulose

On étudie la hauteur de l'interphase dans des tubes de sédimentation, en fonction du temps pour des suspensions dans une solution de nitrocellulose (type CA4 SNPE) à 20 % en poids dans l'acétate de butyle, ce qui reflète la vitesse de sédimentation des particules solides.

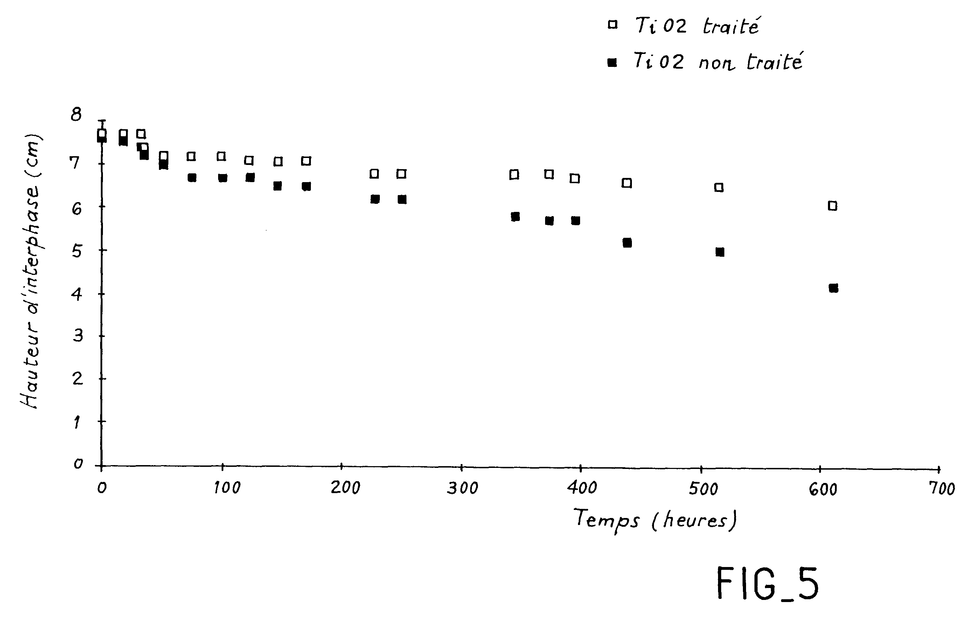

La figure 5 permet de comparer le comportement du TiO$_2$ non traité avec le TiO$_2$ traité par un copolymère Mn=45 000 contenant 18 % en masse de PAA (pour le traitement Ci=3 %).

Exemple 7 : Mise en évidence du rôle dispersant et stabilisant des copolymères PAA-b-PMMA vis-à-vis du $TiO_2$ (S=10 $m^2$/g)

Le tableau ci-dessous rassemble les résultats de dispersion et de stabilisation obtenus pour le $TiO_2$ (S=10 $m^2$/g) et une série de copolymères PAA-b-PMMA (Ci=3 %).

Les agrégats sont constitués des particules dont la taille est supérieure à 1 μm. Dans le cas du $TiO_2$ brut les agrégats représentent 88,4 % en volume.

L'indice Shimadzu est le temps nécessaire (en seconde) pour passer d'une absorbance de 100 % à une absorbance de 50 %. Pour référence, cet indice est de 15 pour le $TiO_2$ non traité.

| Mn total PAA–b–PMMA | % poids PAA | Ci=3 % pour le traitement du $TiO_2$ | |
|---|---|---|---|
| | | dispersion % d'agrégats (en vol) | stabilisation indice Shimadzu |
| 2 300 | 34,2 | 0 | / |
| 9 900 | 22 | 0 | 600 |
| 16 500 | 22,5 | 0 | 532 |
| 17 900 | 32,3 | 0 | 530 |
| 18 900 | 36 | 18,4 | 300 |
| 29 800 | 14,5 | 0 | 463 |
| 31 100 | 35 | 0 | 435 |
| 34 800 | 25,8 | 31,7 | 194 |
| 52 400 | 26,7 | 0 | 607 |
| 57 000 | 31,8 | 35,2 | 200 |
| 59 700 | 49,7 | 23,2 | 233 |

Exemple 8 : Mise en évidence de l'effet dispersant des copolymères dans différents milieux

On étudie successivement la dispersion de deux pigments constitués respectivement d'une poudre d'oxyde de fer jaune de granulométrie moyenne de 0,4 μm et de surface spécifique de 20 $m^2$/g et d'une poudre d'oxyde de titane de 10 $m^2$/g de surface spécifique, en réalisant pour chacun des deux pigments la dispersion des 4 mélanges ci-dessous dans un broyeur à billes, de type Red Devil, utilisé en peinture, pendant une heure.

Les mélanges réalisés sont les suivants, les pourcentages sont donnés en poids :

- mélange 1 (comparatif) :

  . pigment : 20 %
  . acétate de butyle : 80 %

- mélange 2 :

  . pigment 20 %
  . copolymère de l'exemple 1 : 5 %
  . acétate de butyle : 75 %

- mélange 3 :

  . pigment : 20 %
  . copolymère de l'exemple 1 : 5 %

- adjuvant de broyage (Néocryl B 1000) : 5 %
- acétate de butyle : 70 %

- mélange 4

  . pigment : 20 %
  . copolymère de l'exemple 1 : 5 %
  . nitrocellulose : 5 %
  . acétate de butyle : 70 %

Les différents mélanges sont observés au microscope optique et montrent, pour les deux pigments étudiés, une meilleure dispersion des mélanges 2 à 4 par rapport au mélange 1.

Exemple 9 : Préparation d'un vernis à ongles

On utilise des bases incolores de composition suivante :

- nitrocellulose        10 à 15 %
- résine garnissante : Arylsulfamide        8 à 12 %
- plastifiant : dibutylphtalate, camphre        6 à 8 %
- solvants : acétate d'éthyle, acétate de butyle, toluène        65 à 75 %
- agent antisédimentant : bentone        0,8 à 1,5 %
- additifs        1 à 2 %

On prépare des vernis à ongles en procédant de la façon suivante à partir de l'un des mélanges 2 à 4 de l'exemple 8 :

- on dilue le mélange à l'aide d'une base incolore ci-dessus jusqu'à obtention d'une solution colorante contenant environ 5 % en poids de pigment,
- on mélange ensuite le produit obtenu avec une base incolore ci-dessus dans des proportions en fonction de la teinte désirée pour obtenir environ 1 % en poids de pigment dans le vernis terminé.

**Revendications**

1. Utilisation des copolymères-blocs symbolisés par AB de l'acide acrylique ou méthacrylique, le(s) bloc(s) correspondant(s) étant symbolisé(s) par A et de l'acrylate ou du méthacrylate d'alkyle avec la chaîne d'alkyle comprise entre $C_1$ et $C_{10}$, le(s) bloc(s) correspondant(s) étant symbolisé(s) par B, et dans lesquels le bloc A représente moins de 50 % en poids dudit copolymère-bloc, comme agents mouillants et/ou dispersants des particules solides dans un milieu contenant moins de 5 % d'eau et, de préférence moins de 1 % d'eau, constitué d'un solvant ou mélange de solvants solubilisant le bloc B.

2. Utilisation selon la revendication 1, **caractérisée en ce que** lesdits copolymères-blocs sont des copolymères diblocs (A-b-B) ou triblocs (A-b-B-b-A ou B-b-A-b-B).

3. Utilisation selon l'une des revendications 1 ou 2, **caractérisée en ce que** lesdits copolymères-blocs sont des copolymères diblocs de l'acide acrylique (symbolisé par AA) et du méthacrylate de méthyle (symbolisé par MMA) représentés par PAA-b-PMMA.

4. Utilisation selon l'une des revendications 1 à 3, **caractérisée en ce que** les particules ont des dimensions comprises entre quelques nanomètres et quelques millimètres, de préférence entre 50 nm et 100 μm.

5. Utilisation selon l'une des revendications 1 à 4, **caractérisée en ce que** lesdites particules sont des particules minérales, en particulier des oxydes métalliques.

6. Utilisation selon l'une des revendications 1 à 5, **caractérisée en ce que** lesdites particules sont des pigments fixés ou non sur un support organique ou minéral.

**7.** Utilisation selon l'une des revendications 1 à 6, **caractérisée en ce que** lesdites particules sont constituées d'oxyde de titane ($TiO_2$).

**8.** Utilisation selon l'une des revendications 1 à 7, **caractérisée en ce que** le copolymère a une masse molaire moyenne en nombre ($M_n$) comprise entre 2 000 et 500 000, de préférence entre 2 000 et 100 000.

**9.** Utilisation selon l'une des revendications 1 à 8, **caractérisée en ce que** le bloc A, en particulier PAA, dudit copolymère représente 5 à 95 % en poids dudit copolymère.

**10.** Procédé pour préparer une dispersion stable de particules solides en présence d'un agent dispersant dans un milieu essentiellement organique contenant moins de 5 % et de préférence moins de 1 % d'eau, **caractérisé en ce qu'**on utilise à titre d'agent dispersant un copolymère-bloc du type AB tel que défini dans la revendication 1 et, à titre de milieu essentiellement organique, un solvant ou un mélange de solvants, solvant dudit bloc B.

**11.** Procédé selon la revendication 10, **caractérisé en ce que** ledit copolymère bloc est un copolymère de l'acide acrylique (symbolisé par AA) et du méthacrylate de méthyl (symbolisé par MMA).

**12.** Procédé selon la revendication 10 ou 11, **caractérisé en ce que** lesdites particules ont des dimensions comprises entre quelques nanomètres et quelques millimètres, de préférence entre 50 nm et 100 μm.

**13.** Procédé selon l'une des revendications 10 à 12, **caractérisé en ce que** lesdites particules sont des particules minérales, en particulier des oxydes métalliques.

**14.** Procédé selon l'une des revendications 10 à 13, **caractérisé en ce que** lesdites particules sont des pigments fixés ou non sur un support organique ou minéral.

**15.** Procédé selon l'une des revendications 10 à 14, **caractérisé en ce que** lesdites particules sont constituées d'oxyde de titane.

**16.** Procédé selon l'une des revendications 10 à 15, **caractérisé en ce que** le copolymère-bloc a une masse molaire moyenne en nombre comprise entre 2 000 et 500 000, de préférence entre 2 000 et 100 000.

**17.** Procédé selon l'une des revendications 10 à 16, **caractérisé en ce que** la concentration en copolymères-blocs est comprise entre 0,1 et 30 % en poids de copolymère par rapport aux particules solides.

**18.** Procédé selon l'une des revendications 10 à 17, **caractérisé en ce que** le solvant ou le mélange de solvant qui solubilise la séquence B du copolymère est choisi dans le groupe constitué des esters, par exemple, l'acétate de butyle, l'acétgate de propyle, l'acétate d'éthyle ou les mélanges esters/alcools constitués majoritairement d'ester.

**19.** Procédé selon l'une des revendications 10 à 18, **caractérisé en ce que** ledit solvant est de l'acétate de butyle, ou un mélange acétate de buytyle/éthanol contenant au moins 50 % d'acétate de butyle.

**20.** Procédé selon l'une des revendications 10 à 19, **caractérisé en ce qu'**il s'agit d'une dispersion d'oxyde de titane.

**21.** Composition cosmétique contenant une dispersion telle qu'obtenue selon le procédé de l'une des revendications 10 à 20.

**22.** Vernis à ongles **caractérisé en ce qu'**il contient une dispersion telle qu'obtenue selon le procédé de l'une des revendications 10 à 20.

**23.** Composition selon la revendication 21 ou 22, **caractérisée en ce qu'**elle contient, en outre, de la nitrocellulose.

**Patentansprüche**

**1.** Verwendung von durch AB symbolisierten Blockcopolymeren von Acrylsäure oder Methacrylsäure, wobei der entsprechende Block oder die entsprechenden Blöcke durch A symbolisiert wird bzw. werden, und von Alkylacrylat oder -methacrylat, wobei die Alkylkette zwischen $C_1$ und $C_{10}$ umfasst, wobei der entsprechende Block oder die entsprechenden Blöcke durch B symbolisiert wird bzw. werden, und in welchen der Block A weniger als 50 Gew.-% des Blockcopolymers ausmacht, als Benetzungsmittel und/oder Dispergiermittel von festen Teilchen in einem Medium, das weniger als 5% Wasser und vorzugsweise weniger als 1% Wasser enthält und aus einem Lösemittel oder einer Mischung von Lösemitteln, das bzw. die den Block B solubilisiert, gebildet wird.

2. Verwendung nach Anspruch 1, **dadurch gekennzeichnet, dass** die Blockcopolymere Diblockcopolymere (A-b-B) oder Triblockcopolymere (A-b-B-b-A oder B-b-A-b-B) sind.

3. Verwendung nach einem der Ansprüche 1 oder 2, **dadurch gekennzeichnet, dass** die Blockcopolymere Diblockcopolymere von Acrylsäure (symbolisiert durch AA) und Methylmethacrylat (symbolisiert durch MMA) sind, die durch PAA-b-PMMA repräsentiert werden.

4. Verwendung nach einem der Ansprüche 1 bis 3, **dadurch gekennzeichnet, dass** die Teilchen Abmessungen zwischen einigen Nanometern und einigen Millimetern, vorzugsweise zwischen 50 nm und 100 µm haben.

5. Verwendung nach einem der Ansprüche 1 bis 4, **dadurch gekennzeichnet, dass** die Teilchen anorganische Teilchen, insbesondere Metalloxide sind.

6. Verwendung nach einem der Ansprüche 1 bis 5, **dadurch gekennzeichnet, dass** die Teilchen Pigmente sind, die gegebenenfalls auf einem organischen oder anorganischen Träger fixiert sind.

7. Verwendung nach einem der Ansprüche 1 bis 6, **dadurch gekennzeichnet, dass** die Teilchen aus Titanoxid ($TiO_2$) gebildet werden.

8. Verwendung nach einem der Ansprüche 1 bis 7, **dadurch gekennzeichnet, dass** das Copolymer ein MolekulargewichtsZahlenmittel ($M_n$) zwischen 2000 und 500000, vorzugsweise zwischen 2000 und 100000 hat.

9. Verwendung nach einem der Ansprüche 1 bis 8, **dadurch gekennzeichnet, dass** der Block A, insbesondere PAA, des Copolymers 5 bis 95 Gew.-% des Copolymers ausmacht.

10. Verfahren zum Herstellen einer stabilen Dispersion von festen Teilchen in Gegenwart eines Dispergiermittels in einem im wesentlichen organischen Medium, das weniger als 5% und vorzugsweise weniger als 1% Wasser enthält, **dadurch gekennzeichnet, dass** man als Dispergiermittel ein Blockcopolymer vom Typ AB, wie in Anspruch 1 definiert, und als im wesentlichen organisches Medium ein Lösemittel oder eine Mischung von Lösemitteln, das bzw. die ein Lösemittel für den genannten Block B darstellt, verwendet.

11. Verfahren nach Anspruch 10, **dadurch gekennzeichnet, dass** das Blockcopolymer ein Copolymer von Acrylsäure (symbolisiert durch AA) und von Methylmethacrylat (symbolisiert durch MMA) ist.

12. Verfahren nach Anspruch 10 oder 11, **dadurch gekennzeichnet, dass** die Teilchen Abmessungen zwischen einigen Nanometern und einigen Millimetern, vorzugsweise zwischen 50 nm und 100 µm aufweisen.

13. Verfahren nach einem der Ansprüche 10 bis 12, **dadurch gekennzeichnet, dass** die Teilchen anorganische Teilchen, insbesondere Metalloxide, sind.

14. Verfahren nach einem der Ansprüche 10 bis 13, **dadurch gekennzeichnet, dass** die Teilchen Pigmente sind, die gegebenenfalls auf einem organischen oder anorganischen Träger fixiert sind.

15. Verfahren nach einem der Ansprüche 10 bis 14, **dadurch gekennzeichnet, dass** die Teilchen aus Titanoxid gebildet werden.

16. Verfahren nach einem der Ansprüche 10 bis 15, **dadurch gekennzeichnet, dass** das Blockcopolymer ein Molekulargewichts-Zahlenmittel zwischen 2000 und 500000, vorzugsweise zwischen 2000 und 100000 hat.

17. Verfahren nach einem der Ansprüche 10 bis 16, **dadurch gekennzeichnet, dass** die Konzentration an Blockcopolymeren zwischen 0,1 und 30 Gew.-% an Copolymer bezogen auf die festen Teilchen liegt.

18. Verfahren nach einem der Ansprüche 10 bis 17, **dadurch gekennzeichnet, dass** das Lösemittel oder die Mischung von Lösemitteln, das bzw. die die Sequenz B des Copolymers solubilisiert, ausgewählt wird in der Gruppe, gebildet aus Estern, beispielsweise Butylacetat, Propylacetat, Ethylacetat, oder den Mischungen von Estern und Alkoholen, die überwiegend aus Ester gebildet werden.

19. Verfahren nach einem der Ansprüche 10 bis 18, **dadurch gekennzeichnet, dass** das Lösemittel Butylacetat oder eine Mischung von Butylacetat und Ethanol, die mindestens 50% Butylacetat enthält, ist.

20. Verfahren nach einem der Ansprüche 10 bis 19, **dadurch gekennzeichnet, dass** es sich um eine Titanoxiddispersion handelt.

21. Kosmetische Zusammensetzung, die eine Dispersion, wie sie gemäß dem Verfahren von einem der Ansprüche 10 bis 20 erhalten wird, enthält.

22. Nagellack, **dadurch gekennzeichnet, dass** er eine Dispersion, wie sie gemäß dem Verfahren von einem der Ansprüche 10 bis 20 erhalten wird, enthält.

23. Zusammensetzung nach Anspruch 21 oder 22, **dadurch gekennzeichnet, dass** sie ausserdem Nitrocellulose enthält.

**Claims**

1. A use of block copolymers, symbolized by AB, of acrylic or methacrylic acid, the corresponding block(s) being symbolized by A, and of alkyl acrylate or methacrylate with the alkyl chain being between $C_1$ and $C_{10}$, the corresponding block(s) being symbolized by B, wherein the block A represents less than 50% by weight of said block copolymer, as wetting and/or dispersing agents for solid particles in an essentially organic medium containing less than 5% water and, preferably, less than 1% water, consisting of a solvent or solvent mixture solubilizing the block B.

2. The use according to claim 1 **characterized in that** said block copolymers are two-block copolymers (A-b-B) or three-block copolymers (A-b-B-b-A or B-b-A-b-B).

3. The use according to one of claims 1 or 2 **characterized in that** said block copolymers are two-block copolymers of acrylic acid (symbolized by AA) and methyl methacrylate (symbolized by MMA), represented by PAA-b-PMMA.

4. The use according to claims 1 to 3 **characterized in that** the particles have dimensions of between a few nanometers and a few millimeters, preferably of between 50 nm and 100 μm.

5. The use according to one of claims 1 to 4 **characterized in that** said particles are mineral particles, particularly metal oxide particles.

6. The use according to one of claims 1 to 5 **characterized in that** said particles are pigments which may or may not be fixed to an organic or mineral support.

7. The use according to one of claims 1 to 6 **characterized in that** said particles consist of titanium oxide ($TiO_2$).

8. The use according to one of claims 1 to 7 **characterized in that** the copolymer has a number-average molecular weight ($M_n$) of between 2000 and 500,000, preferably of between 2000 and 100,000.

9. The use according to one of claims 1 to 8 **characterized in that** the block A, particularly PAA, of said copolymer represents 5 to 95% by weight of said copolymer.

10. A process for preparing a stable dispersion of solid particles in the presence of a dispersing agent in an essentially organic medium containing less than 5%, preferably less than 1% water, **characterized in that** as the dispersing agent a block copolymer of the AB type as defined in claim 1, and as an essentially organic medium, a solvent or a solvent mixture solubilizing said block B, are used.

11. The process according to claim 10 **characterized in that** said block copolymer is a block copolymer of acrylic acid (being symbolized by AA) and of methyl methacrylate (being symbolized by MMA).

**12.** The process according to claim 10 or 11 **characterized in that** said particles have dimensions of between a few nanometers and a few millimeters, preferably of between 50 nm and 100 μm.

**13.** The process according to one of claims 10 to 12 **characterized in that** said particles are mineral particles, particularly metal oxide particles.

**14.** The process according to one of claims 10 to 13 **characterized in that** said particles are pigments which may or may not be fixed to an organic or mineral support.

**15.** The process according to one of claims 10 to 14 **characterized in that** said particles consist of titanium oxide.

**16.** The process according to one of claims 10 to 15 **characterized in that** the block copolymer has a number-average molecular weight of between 2000 and 500,000, preferably of between 2000 and 100,000.

**17.** The process according to one of claims 10 to 16 **characterized in that** the concentration of block copolymers is between 0.1 and 30% by weight of copolymer, based on the solid particles.

**18.** The process according to one of claims 10 to 17 **characterized in that** the solvent or solvent mixture which solubilizes the block B of the copolymer is selected from the group consisting of esters, for example butyl acetate, propyl acetate or ethyl acetate, and ester/alcohol mixtures consisting predominantly of ester.

**19.** The method according to one of claims 10 to 18 **characterized in that** said solvent is butyl acetate or a butyl acetate/ethanol mixture containing at least 50% of butyl acetate.

**20.** The process according to one of claims 10 to 19 **characterized in that** said dispersion is a titanium oxide dispersion.

**21.** A cosmetic composition as prepared according to the process of one of claims 10 to 20.

**22.** A nail varnish **characterized in that** it contains a dispersion as prepared according to the process of one of claims 10 to 20.

**23.** A composition according to claim 21 or 22 **characterized in that** it also contains nitrocellulose.

FIG_1

TiO2 dans l'AcBu

FIG_2a

Ci = 1%

FIG_2b

Ci = 3%

FIG_2c

FIG_3

FIG_4

FIG_5

EP 0 721 486 B1